# EUROPÄISCHE PATENTSCHRIFT

(11) **EP 1 236 685 B1**
(45) Veröffentlichungstag und Bekanntmachung des Hinweises auf die Patenterteilung: **08.08.2007**
(21) Anmeldenummer: 02000892.6
(22) Anmeldetag: 15.01.2002
(51) Int. Cl.: C02F 1/44, A61M 1/16, B01D 61/08, B01D 65/02, A61L 2/04, B01D 61/10

(54) **Reinstwasser-Versorgungsanlage und Verfahren zur termischen Desinfektion des Reinstwasser-Versorgungssystems von Dialysegeräten**
Ultrapure water supply system and method for heat disinfecting the ultrapure water supply system of dialyzers
Système d'alimentation d'eau ultra-pure et procédé de désinfection thermique de systèmes d'alimentation d'eau ultra-pure de dialyseurs

(30) Priorität: 02.03.2001 DE 10110188; 08.03.2001 DE 10111104
(43) Veröffentlichungstag der Anmeldung: 04.09.2002
(73) Patentinhaber: Völker, Manfred, 63825 Blankenbach (DE)
(72) Erfinder: Völker, Manfred, 63825 Blankenbach (DE); Schäl, Wilfried, 61350 Bad-Homburg (DE)
(74) Vertreter: Flosdorff, Jürgen

(56) Entgegenhaltungen:
- WO-A-01/41895
- DE-A- 10 013 964
- DE-A- 19 538 818
- DE-A- 19 933 223
- US-A- 5 591 344

## Beschreibung

Die Erfindung betrifft eine Reinstwasser-Versorgungsanlage für wenigstens ein, vorzugsweise mehrere Dialysegeräte einer Dialysestation, mit einem Umkehrosmosegerät, das Reinstwasser einer Reinstwasser-Versorgungsleitung zuführt, von der Anschlußleitungen zu den Dialysegeräten abzweigen. Die Erfindung betrifft ferner ein Verfahren zur thermischen Desinfektion der Reinstwasser-Versorgungsanlage der Dialysegeräte einer Dialysestation.

Bei Hämodialysegeräten hat sich seit langem die Heißdesinfektion/Heißreinigung gut bewährt.

DE 195 38 818 A1 offenbart eine Anlage zur Versorgung einer Dialysestation mit Dialysewasser, die eine Ringleitung, an welche die Dialysestation angeschlossen ist, einen Umkehrosmosemodul zur Herstellung des Dialysewassers, welches in die Ringleitung eingeleitet wird, eine Zirkulationspumpe, einen Ultrafiltrationsmodul, sowie eine Heizeinrichtung enthält, die zur thermischen Desinfektion das zirkulierende Reinstwasser des gesamten Versorgungsleitungssystems aufheizt. Die vorbekannte Anlage hat den Nachteil, dass zum Aufheizen des Leitungssystems kurzzeitig große Energiemengen bereit gestellt werden müssen.

DE 199 33 223 A1 offenbart ein Verfahren zur thermischen Desinfektion von Hämodialyseanlagen, bei dem die Heizvorrichtungen der Hämodialysegeräte dazu herangezogen werden, den gesamten Wasserkreislauf aufzuheizen, anstatt hierzu eine zentrale Heizvorrichtung vorzusehen.

US-A-5,591,344 offenbart einen mit der Versorgungsleitung in Verbindung stehenden Tank, in dem Chemikalien dem Wasser beigemischt werden. WO01/41895 A, fallend unter Artikel 54 (3) EPÜ, offenbart einen Reinstwasserspeichertank, der stromabwärts eines Umkehrosmosegeräts angeordnet ist und von diesem mit Reinstwasser befüllt wird, das von einer Pumpe zu einer Dialysestation gepumpt wird.

Der vorliegenden Erfindung liegt die Aufgabe zugrunde, einen stoßartig hohen Energieverbrauch bei der thermischen Desinfektion zu vermeiden.

Diese Aufgabe wird erfindungsgemäß durch die Merkmale der Patentansprüche 1 und 2 gelöst.

Vorteilhafte Ausgestaltungen der Erfindung sind in den abhängigen Ansprüchen gekennzeichnet.

Die erfindungsgemäße Reinstwasser-Versorgungsanlage enthält einen Heißwasserspeicher, der mit der als Ringleitung ausgebildeten Versorgungsleitung in Verbindung steht. Während die kurzzeitige Bereitstellung großer Energiemengen, wie sie zum Aufheizen des Leitungssystems erforderlich wären, in der Praxis oft auf das Problem stößt, daß entsprechend leistungsfähige Elektroanschlüsse häufig nicht zur Verfügung gestellt werden können, weshalb diese Lösung des Problems für die Praxis ungeeignet ist, steht für das Aufheizen des erfindungsgemäß vorgesehenen Heißwasserspeichers eine relativ lange Zeitspanne zur Verfügung, so daß ein stoßartiger hoher Energieverbrauch vermieden wird.

Hierzu sind erfindungsgemäß ein Heizaggregat und eine Zirkulationspumpe für die thermische Desinfektion vorgesehen, wobei nach einem weiteren wesentlichen Merkmal der Erfindung vorgeschlagen wird, daß sich diese für die thermische Desinfektion erforderlichen Zusatzeinrichtungen stromabwärts der Anschlußleitungen der Dialysegeräte in die Reinstwasser-Versorgungsleitung einfügen. Hierdurch wird erreicht, daß die Dialysegeräte im normalen Betrieb weiterhin direkt aus der Ringleitung, ohne den Umweg über die für die thermische Desinfektion vorgesehenen Zusatzeinrichtungen mit Reinstwasser versorgt werden. Da der Heißwasserspeicher, das Heizaggregat und die Zirkulationspumpe stromabwärts der Entnahmestellen des Reinstwassers für die Dialysegeräte liegen, können diese Zusatzeinrichtungen keine Kontaminationsquellen für das gelieferte Permeat bilden.

Weiter ist erfindungsgemäß vorgesehen, daß von dem hinter den Anschlußleitungen der Dialysegeräte liegenden Rücklaufabschnitt der Ringleitung eine zum Vorlaufabschnitt zurückführende Verbindungsleitung abzweigt, mit der der Heißwasserspeicher über eine Kreislaufleitung in Verbindung steht. In diese Kreislaufleitung sind außerdem das Heizaggregat und die Zirkulationspumpe integriert.

Das erfindungsgemäße Verfahren zur thermischen Desinfektion der Reinwasserversorgung des Reinstwasser-Versorgungssystems sieht vor, daß der Heißwasserspeicher über die Ringleitung mit in dem Umkehrosmosegerät aufbereiteten Reinstwasser befüllt wird, daß das Reinstwasser dann in einem geschlossenen Kreislauf auf eine vorbestimmte Heißreinigungstemperatur erhitzt wird und daß die Ringleitung dann mit dem Heißwasser durchströmt wird, bis die Temperatur am Endbereich des Rücklaufabschnitts der Ringleitung einen vorbestimmten Mindestwert erreicht.

Weiter ist vorgesehen, daß das Heißwasser anschließend in der Ringleitung zirkuliert, wobei von der Steuerungseinrichtung des Umkehrosmosegerätes ein Signal an die angeschlossenen Dialysegeräte abgegeben wird, so daß diese Heißwasser aus der Ringleitung entnehmen. Damit werden auch die Anschlußleitungen zwischen der Ringleitung und den Dialysegeräten in die thermische Desinfektion einbezogen.

Anschließend kann die Zirkulation des Heißwassers in der Ringleitung über eine einstellbare Zeitspanne fortgesetzt werden.

Die thermische Desinfektion endet damit, daß die Ringleitung mit dem aufbereiteten Reinstwasser des Umkehrosmosegerätes gespeist wird, bis das Heißwasser vollständig verdrängt wird. Dabei wird das gesamte zur Heißreinigung benutzte Wasser ausgespült und die Ringleitung gekühlt.

Außerdem können alle vor dem Filtermodul des Umkehrosmosegerätes liegenden Strömungswege bis hin zum Einlaßventil und das Filtermodul selbst in die Heißreinigung einbezogen werden, wie weiter unten in näheren Einzelheiten beschrieben wird.

Mit der vorliegenden Erfindung läßt sich durch weitgehende Automatisierung des Reinigungsablaufs eine erhebliche Arbeitsvereinfachung erzielen. Das Reinigungsprogramm kann an vorwählbaren Wochentagen automatisch im Anschluß an die normale Dialyse-Versorgungsperiode gestartet werden, woraufhin das Reinigungsverfahren vollkommen selbständig abläuft. Bei Bedarf kann das Reinigungsprogramm auch jederzeit manuell gestartet werden.

Die Funktionsweise des thermischen Reinigungs-/Desinfektionsverfahrens ist aus den beigefügten schematischen Darstellungen der erfindungsgemäßen Reinwasser-Versorgungsanlage ersichtlich. Dabei zeigen:
- Figur 1: den Verfahrensschritt des Füllens des Wärmespeichers;
- Figur 2: den Verfahrensschritt des Aufheizens des Wärmespeichers;
- Figur 3: den Verfahrensschritt des Heißspülens der Ringleitung;
- Figur 4: den Verfahrensschritt der Zirkulation mit Entnahme;
- Figur 5: den Verfahrensschritt der Zirkulation ohne Entnahme und
- Figur 6: den Verfahrensschritt des Kühlens der Ringleitung,

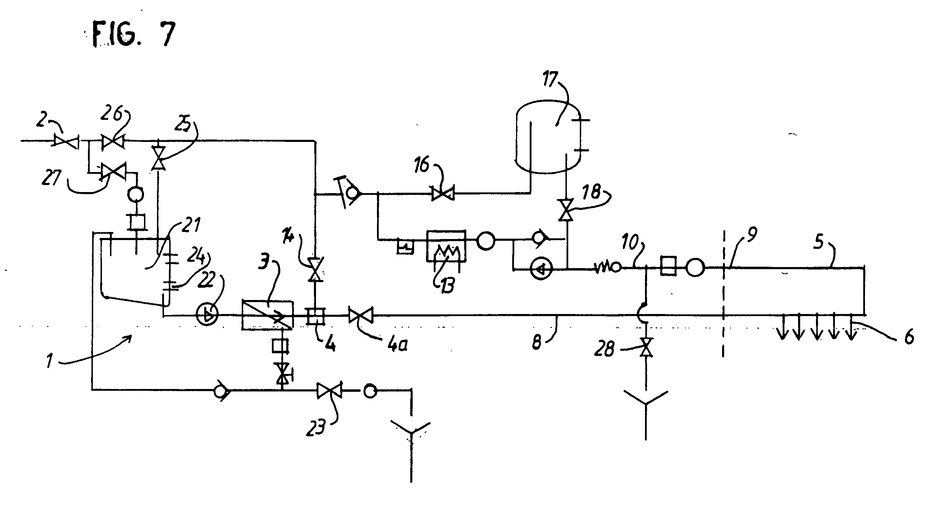

- Figur 7: die Einbeziehung der Strömungswege und des Filtermoduls des Umkehrosmosegerätes in die Heißreinigung.

In den Figuren ist rein schematisch ein Umkehrosmosegerät 1 dargestellt, in das über einen Einlaß 2 Wasser eingeführt wird, das in dem Umkehrosmosegerät 1 zu Reinstwasser (Permeat) aufbereitet wird. Das Permeat gelangt durch einen Filter 3 in dem Umkehrosmosegerät 1, anschließend über eine Überwachungseinrichtung 4, die den Leitfähigkeitswert und die Temperatur des Permeats überwacht, und ein Freigabeventil 4a in eine sogenannte Ringleitung 5, von der Anschlußleitungen 6 zu den jeweils zugehörigen Dialysegeräten 7 abzweigen.

Die Ringleitung 5 läßt sich in einen bis zu den Anschlußleitungen 6 reichenden Vorlaufabschnitt 8 und einen anschließenden Rücklaufabschnitt 9 unterteilen.

Stromabwärts der Anschlußleitungen 6 der Dialysegeräte 7 zweigt eine Verbindungsleitung 10 ab, die zum Freigabeventil bzw. Einlaßventil 4 des Vorlaufabschnitts 8 führt. In diese Verbindungsleitung 10 sind nacheinander ein Rückschlag- bzw. Einwegventil 11, eine Zirkulationspumpe 12, ein elektrisches Heizaggregat 13 und ein Bypassventil 14 integriert.

Stromabwärts des Heizaggregats 13 zweigt eine Kreislaufleitung 15 mit einem Ventil 16 ab, die in einen Wärmespeicher 17 für Heißwasser einmündet, von dem die Kreislaufleitung 15 zur Verbindungsleitung 10 - zwischen dem Einwegventil 11 und der Zirkulationspumpe 12 - zurückführt. Im Bereich der Einmündung ist ein Ventil 18 in die Kreislaufleitung 15 integriert.

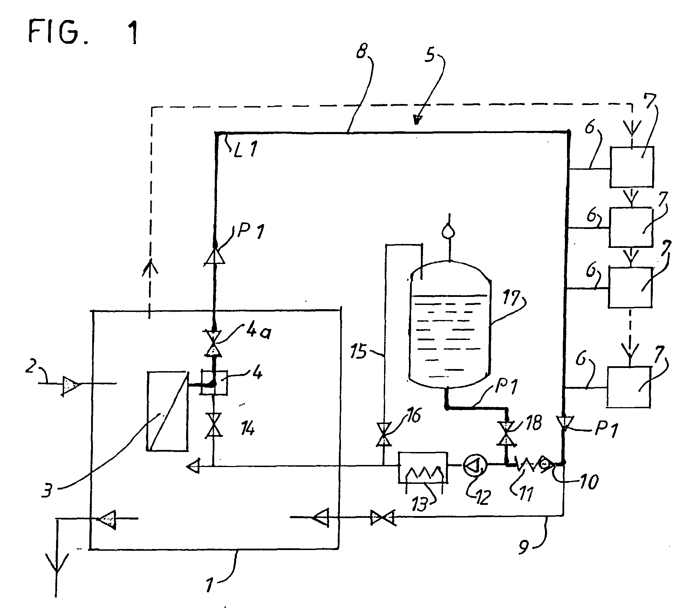

Bei dem ersten Verfahrensschritt des Füllens des Wärmespeichers 17 wird dieser über die Ringleitung 5, die Verbindungsleitung 10 und die Kreislaufleitung 15 bis zu einem vorgegebenen Füllstand befüllt, was durch eine mit Pfeilen P1 versehene Linie L1 in Figur 1 angedeutet ist.

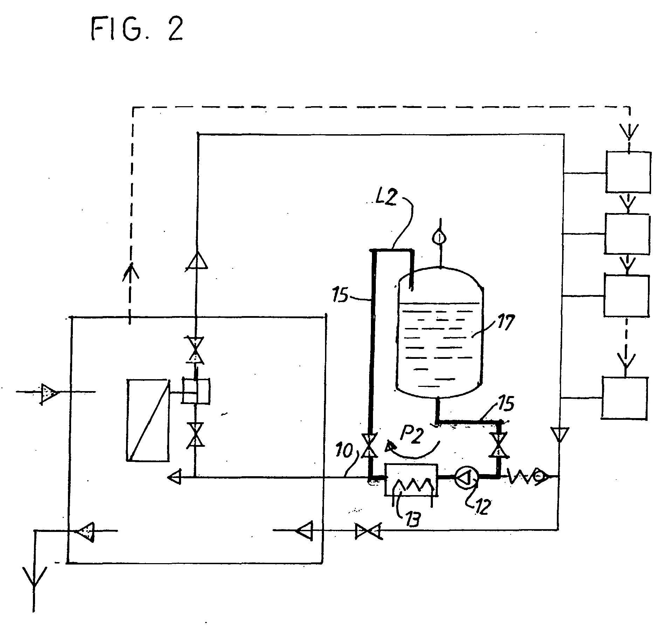

Nach Erreichen des vorgesehenen Füllstands wird das Permeat im geschlossenen Kreislauf, bestehend aus dem Wärmespeicher 17, der Zirkulationspumpe 12 und dem Heizaggregat 13 auf die vorgesehene Heißreinigungstemperatur erhitzt. Der geschlossene Kreislauf ist durch die Linie L2 in Figur 2 angedeutet. Das Permeat zirkuliert in Richtung des Pfeils P2.

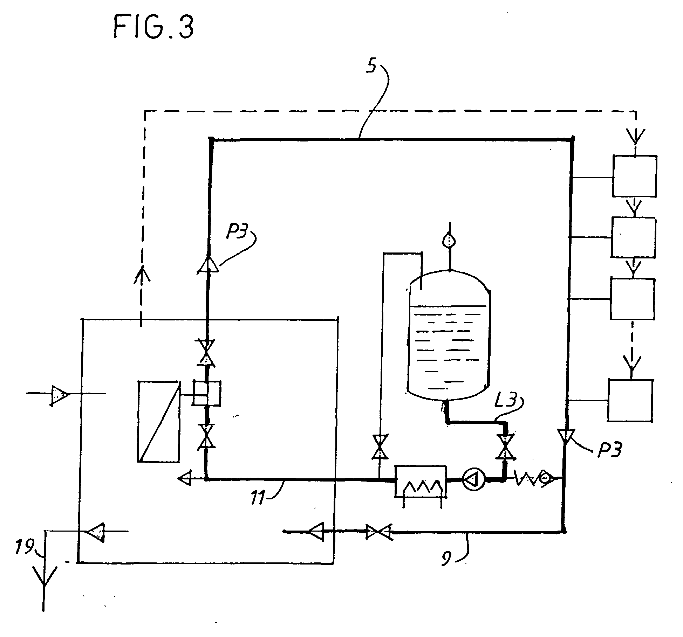

Im nächsten Verfahrensschritt wird - wie Figur 3 zeigt - das erhitzte Permeat entlang der Linie L3 durch den unteren Zweig der Kreislaufleitung 15, den anschließenden Abschnitt der Verbindungsleitung 11 und die Ringleitung 5 gepumpt, wobei das Heißwasser den Rücklaufabschnitt 9 der Ringleitung 5 durchströmt und das Versorgungssystem aus seinem Auslaß 19 verläßt. Die Ringleitung 5 wird auf diese Weise so lange mit Heißwasser durchströmt, bis die Temperatur am Ende der Ringleitung 5 den vorgesehenen Mindestwert erreicht hat.

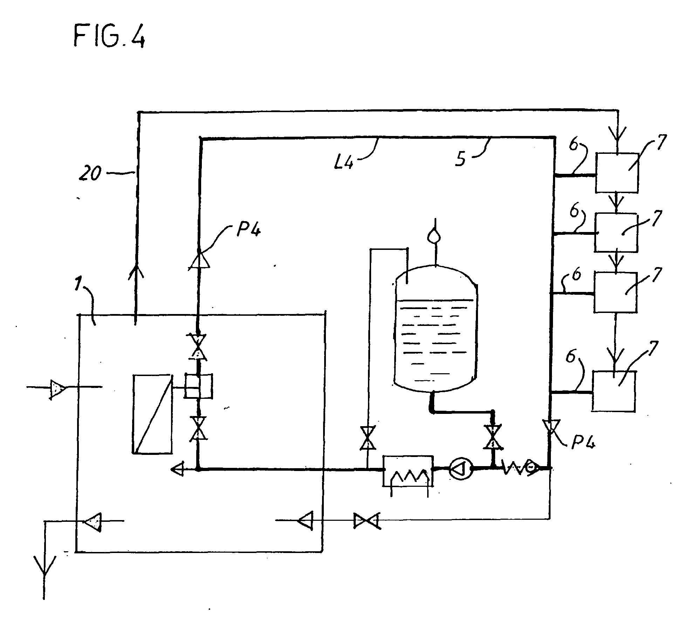

Im nächsten Verfahrensschritt, der in Figur 4 dargestellt ist, wird das Heißwasser in der Ringleitung 5 zirkuliert, was durch die Linie L4 und die Pfeile P4 angedeutet ist. Dabei gibt die Steuerungseinrichtung des Umkehrosmosegeräts 1 über eine Steuerleitung 20 ein Signal an die anschlossenen Dialysegeräte 7 ab, woraufhin diese durch die Anschlußleitungen 6 Heißwasser aus der Ringleitung 5 entnehmen, womit auch diese Anschlußleitungen 6 zwischen der Ringleitung 5 und den Dialysegeräten 7 in die thermische Desinfektion einbezogen werden.

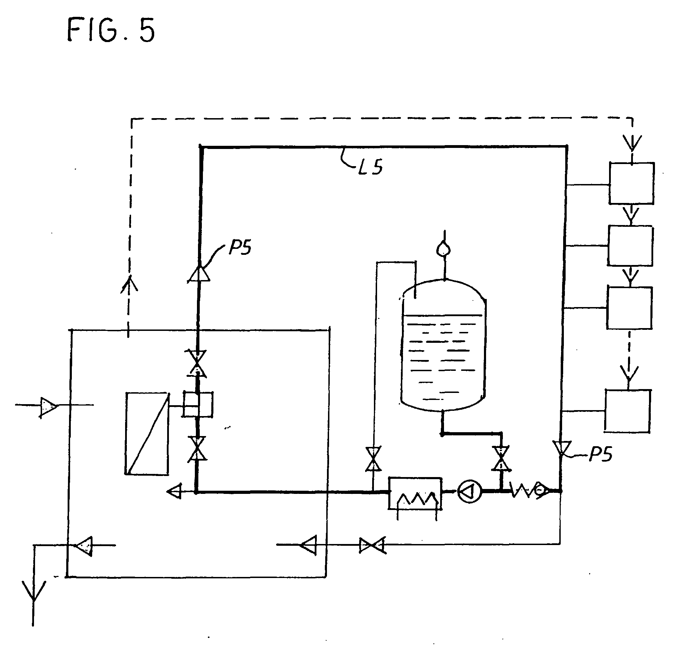

Anschließend kann das Heißwasser in der Ringleitung ohne Entnahme durch die Dialysegeräte 7 für eine einstellbare Zeitspanne weiter zirkulieren, wie Figur 5 mit den Linien L5 und den Pfeilen P5 andeutet.

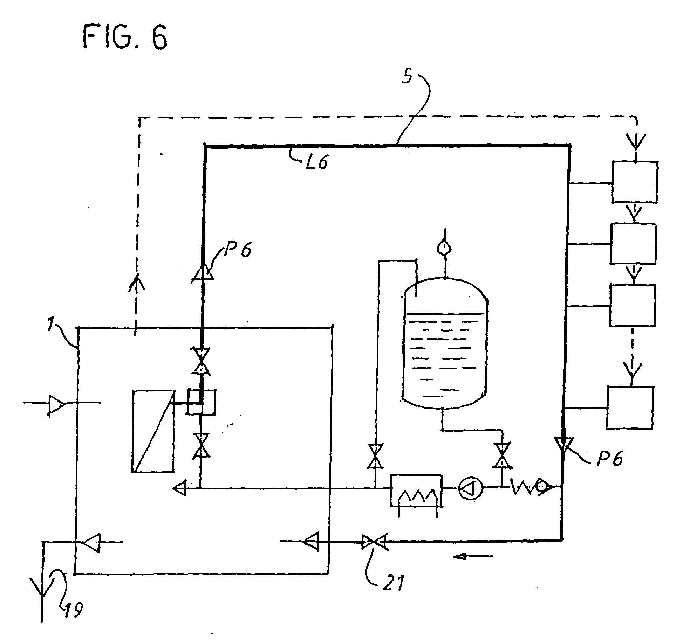

In dem Verfahrensschritt gemäß Figur 6 wird die Ringleitung 5 mit aufbereitetem Reinstwasser (Permeat) aus dem Umkehrosmosegerät 1 gespeist, was durch die Linie L6 und die Pfeile P6 angedeutet ist. Dies erfolgt solange, bis das Heißwasser vollständig aus der Ringleitung 5 verdrängt, d.h. aus dem Auslaß 19 der Ringleitung ausgespült worden ist. Durch das zugeführte Reinstwasser wird die Ringleitung 5 gekühlt, so daß anschließend die normalen Dialysebehandlungen erfolgen können. Es schließen sich das (Nach-)Füllen des Wärmespeichers und dessen (Nach-)Heizen an.

Der Rücklaufabschnitt 9 der Ringleitung 5 enthält ein Ventil 21, mit dem der Rücklaufabschnitt 9 bei Bedarf verschließbar ist.

Das erfindungsgemäße Verfahren ermöglicht die thermische Desinfektion/Reinigung des gesamten Leitungssystems, über das die Dialysegeräte einer Dialysestation von einer zentralen Stelle aus mit aufbereitetem Wasser versorgt werden.

Mit dem erfindungsgemäßen Verfahren werden bevorzugt nicht nur die Ringleitung und die Stichleitungen zu den Dialysegeräten heiß gereinigt, sondern es können zusätzlich alle vor dem Filtermodul 3 des Umkehrosmosegerätes 1 liegenden Strömungswege bis hin zu dem Einlaßventil 2 und das Filtermodul 3 selbst in die Heißreinigung miteinbezogen werden. In diesem Fall werden alle Anlagenteile von der Heißreinigung umfaßt.

Hierzu wird zunächst der Füllstand in dem Vorlaufbehälter 21 des Umkehrosmosegerätes 1 abgesenkt. Dies ist erforderlich, da die Membranen des Umkehrosmosegerätes (RO) nicht sofort mit Heißwasser beaufschlagt werden dürfen. Dem wird dadurch Rechnung getragen, daß das aus dem Wärmespeicher 17 zugeführte Heißwasser durch Vermischen mit dem im Vorlaufbehälter 21 befindlichen Kaltwasser abgekühlt wird.

Bei diesem Verfahrensschritt wird die Pumpe 22 des RO-Gerätes 1 bei geöffnetem Auslaßventil 23 in Betrieb gesetzt. Dieser Vorgang endet, wenn das Niveau 24 in dem Vorlaufbehälter 21 erreicht ist.

Anschließend erfolgt der Betrieb des RO-Gerätes 1 mit Heißwasser. Der Vorlaufbehälter 21 des Gerätes 1 wird mittels der Pumpe 12 über die geöffneten Ventile 18 und 25 aus dem Heißwasserbehälter 17 gespeist. Im Wechsel mit dem Ventil 25 werden auch Ventile 26 und 27 geöffnet, um auch deren Strömungswege einzubeziehen. Die Regelung der Zufuhr zum Vorlaufbehälter 21 erfolgt - in Abhängigkeit von dem dort gemessenen Füllstand - durch zeitweiliges Öffnen des Ventils 16.

Die Pumpe 22 des RO-Gerätes 1 läuft mit minimierter Drehzahl, das Ventil 4a bleibt ständig geschlossen, das Ventil 23 wird zeitweilig geöffnet bzw. geschlossen, um den Füllstand in dem RO-Gerät 1 einzubeziehen. Nach erreichter Temperatur, die an der Überwachungseinrichtung 4 gemessen wird, wird für eine fest eingestellte Zeit das Heißwasser über das RO-Gerät 1 zirkuliert. Nach Abschluß der Zirkulationsphase wird das Ventil 4a geöffnet, ebenso ein Auslaßventil 28, und das restliche Heißwasser wird verbraucht.

## Patentansprüche

1. Reinstwasser-Versorgungsanlage für wenigstens ein, vorzugsweise mehrere Dialysegeräte (7), mit einem Umkehrosmosegerät (1), dessen Permeatausgang zum Zuführen von Reinstwasser mit dem Vorlaufabschnitt (8) einer als Ringleitung (5) ausgebildeten Versorgungsleitung in Verbindung steht, wobei der Vorlaufabschnitt (8) bis zu der von der Ringleitung (5) zu den Dialysegeräten (7) abzweigenden Anschlußleitungen (6) reicht,
**dadurch gekennzeichnet,**
**dass** von dem stromabwärts der Anschlussleitungen (6) der Dialysegeräte (7) liegenden Rücklaufabschnitt (9) der Ringleitung (5) eine zum Vorlaufabschnitt (8) zurückführende Verbindungsleitung (10) abzweigt und
**dass** ein Heißwasserspeicher (17), eine Zirkulationspumpe (12) und ein elektrisches Heizaggregat (13) in einen Kreislauf (15) integriert sind, der von der Verbindungsleitung (10) abzweigt und einen Abschnitt von dieser enthält.

2. Verfahren zur thermischen Desinfektion einer Reinstwasser-Versorgungsanlage nach Anspruch 1,
**dadurch gekennzeichnet,**
**dass** der Heißwasserspeicher (17) über die Ringleitung (5) mit Reinstwasser befüllt wird,
das das Reinstwasser in einem geschlossenen Kreislauf auf eine vorbestimmte Heißreinigungstemperatur erhitzt wird und
**dass** die Ringleitung (5) mit dem Heißwasser durchströmt wird, bis die Temperatur am Endbereich des hinter den Anschlussleitungen (6) der Dialysegeräte (7) liegenden Rücklaufabschnitts der Ringleitung (5) einen vorbestimmten Mindestwert erreicht.

3. Verfahren nach Anspruch 2,
**dadurch gekennzeichnet,**
**daß** anschließend das Heißwasser in der Ringleitung (5) zirkuliert, wobei von der Steuereinrichtung des Umkehrosmosegerätes (1) ein Signal an die angeschlossenen Dialysegeräte (7) abgegeben wird, so dass diese Heißwasser durch ihre Anschlußleitungen (6) aus der Ringleitung (5) entnehmen.

4. Verfahren nach Anspruch 2 oder 3,
**dadurch gekennzeichnet,**
**daß** anschließend die Zirkulation des Heißwassers in der Ringleitung (5) über eine einstellbare Zeitspanne fortgesetzt wird.

5. Verfahren nach einem der Ansprüche 2 bis 4,
**dadurch gekennzeichnet,**
**daß** anschließend die Ringleitung (5) mit aufbereitetem Reinstwasser gespeist wird, bis das Heißwasser vollständig verdrängt und ausgespült ist.

6. Verfahren nach einem der Ansprüche 2 bis 5,
**dadurch gekennzeichnet,**
**dass** der Füllstand in dem Vorlaufbehälter des Umkehrosmosegerätes (1) abgesenkt wird und dass das Umkehrosmosegerät (1) mit Heißwasser gereinigt wird.

## Claims

1. An ultrapure-water supply system for at least one, preferably a plurality of
dialyzers (7), comprising a reverse osmosis device (1) having a permeate outlet which for the supply of ultrapure water is in communication with the inlet section (8) of a supply line configured as a ring line (5), the inlet section (8) extending up to branch lines (6) branching off from the ring line (5) to the dialyzers (7),
**characterized in**
**that** a connection line (10) which leads back to the inlet section (8) branches off from the return section (9) of the ring line (5) which is located downstream of the branch lines (6) of the dialyzers (7), and
**that** a hot water tank (17), a circulation pump (12) and an electrical heating unit (13) are integrated into a circuit (15) which branches off from the connection line (10) and contains a section of said line.

2. A method for thermally disinfecting an ultrapure-water supply system according to claim 1,
**characterized in**
**that** the hot-water tank (17) is filled via the ring line (5) with ultrapure water, that the ultrapure water is heated in a closed circuit to a predetermined hot cleaning temperature, and
**that** the hot water flows through the ring line (5) until the temperature at the end portion of the return section of the ring line (5) located behind the branch lines (6) of the dialyzers (7) reaches a predetermined minimum value.

3. The method according to claim 2,
**characterized in**
**that** the hot water is subsequently circulated in the ring line (5), the control means of the reverse osmosis device (1) outputting a signal to the connected dialyzers (7), so that said dialyzers draw hot water through their branch lines (6) from the ring line (5).

4. The method according to claim 2 or 3,
**characterized in**
**that** subsequently the circulation of the hot water in the ring line (5) is continued for an adjustable period of time.

5. The method according to any one of claims 2 to 4,
**characterized in**
**that** subsequently the ring line (5) is fed with purified ultrapure water until the hot water is completely displaced and flushed out.

6. The method according to any one of claims 2 to 5,
**characterized in**
**that** the fill level in the feed tank of the reverse osmosis device (1) is lowered, and that the reverse osmosis device (1) is purified with hot water.

## Revendications

1. Système d'alimentation en eau ultra-pure pour au moins un, de préférence plusieurs appareils de dialyse (7), avec un appareil d'osmose inverse (1), dont la sortie du perméat se trouve en communication pour l'alimentation en eau ultra-pure avec le tronçon aller (8) d'une conduite d'alimentation configurée comme conduite annulaire (5), sachant que le tronçon aller (8) va jusqu'aux conduites de raccordement (6) bifurquant de la conduite annulaire (5) vers les appareils de dialyse (7),
**caractérisé en ce que**
une conduite de raccordement (10) renvoyant au tronçon aller (8) bifurque depuis la section de retour (9) de la conduite annulaire (5) se trouvant en aval des conduites de raccordement (6) des appareils de dialyse (7) et
**en ce qu'**un accumulateur d'eau chaude (17), une pompe de circulation (12) et un appareil de chauffage électrique (13) sont intégrés dans un circuit (15) qui bifurque de la conduite de raccordement (10) et contient un tronçon de celle-ci.

2. Procédé de désinfection thermique d'un système d'alimentation en eau ultra-pure selon la revendication 1,
**caractérisé en ce que**
l'accumulateur d'eau chaude (17) est rempli d'eau ultra-pure par la conduite annulaire (5),
**en ce que** l'eau ultra-pure est chauffée dans un circuit fermé à une température de nettoyage à chaud prédéterminée et
**en ce que** la conduite annulaire (5) est traversée par l'eau chaude jusqu'à ce que la température dans la zone d'extrémité du tronçon de retour de la conduite annulaire (5) se trouvant en aval des conduites de raccordement (6) des appareils de dialyse (7) atteigne une valeur minimale prédéterminée.

3. Procédé selon la revendication 2,
**caractérisé en ce que**
l'eau chaude circule ensuite dans la conduite annulaire (5), lors de quoi un signal est émis par le dispositif de commande de l'appareil d'osmose inverse (1) aux appareils de dialyse (7) raccordés, de telle sorte que ceux-ci prélèvent de l'eau chaude de la conduite annulaire (5) par leurs conduites de raccordement (6).

4. Procédé selon la revendication 2 ou 3,
**caractérisé en ce que**
la circulation de l'eau chaude dans la conduite annulaire (5) se poursuit ensuite pendant un laps de temps réglable.

5. Procédé selon l'une quelconque des revendications 2 à 4,
**caractérisé en ce que**
la conduite annulaire (5) est ensuite alimentée avec de l'eau ultra-pure traitée, jusqu'à ce que l'eau chaude soit complètement refoulée et éliminée par rinçage.

6. Procédé selon l'une quelconque des revendications 2 à 5,
**caractérisé en ce que** le niveau de remplissage dans le réservoir d'entrée de l'appareil d'osmose inverse (1) est abaissé et **en ce que** l'appareil d'osmose inverse (1) est nettoyé avec de l'eau chaude.
